# EUROPÄISCHE PATENTANMELDUNG

(11) **EP 3 892 245 A1**
(43) Veröffentlichungstag der Anmeldung: **13.10.2021**
(21) Anmeldenummer: 21162034.9
(22) Anmeldetag: 11.03.2021
(51) Int. Cl.: A61F 7/08, A61F 7/02

(54) **WÄRMFLASCHE UND ÜBERZUG EINER WÄRMFLASCHE**

(30) Priorität: 07.04.2020 DE 102020109614
(71) Anmelder: Frosch, Hugo, 86470 Thannhausen (DE)
(72) Erfinder: Frosch, Hugo, 86470 Thannhausen (DE)
(74) Vertreter: Dr. Binder & Binder GbR

(57) **Zusammenfassung**

Die Erfindung betrifft eine Wärmflasche (1), aufweisend einen über eine verschließbare Einfüllöffnung (3) mit einer Flüssigkeit befüllbaren Hohlkörper (2) aus einem Kunststoff, der kein Verbundstoff aus Fasern oder Kunststoff ist, mit einer Innenseite und einer Außenseite, wobei zumindest das die Außenseite bildende Material der Wärmflasche (1) mindestens ein Biozid aufweist. Weiterhin betrifft die Erfindung einen Überzug (4) einer Wärmflasche (1), der zur einmaligen Verwendung ausgebildet ist. Außerdem betrifft die Erfindung die Verwendung eines Kunststoffes mit einem Additiv mit biozider Wirkung zur Herstellung einer Wärmflasche (1).

## Beschreibung

Die Erfindung betrifft eine Wärmflasche, aufweisend einen über eine verschließbare Einfüllöffnung mit einer Flüssigkeit befüllbaren Hohlkörper aus einem Kunststoff mit einer Innenseite und einer Außenseite. Weiterhin betrifft die Erfindung auch die Ausstattung einer solchen Wärmflasche mit einem Überzug auf deren Außenseite. Außerdem betrifft die Erfindung auch die Verwendung eines mit einem Additiv versehenen Kunststoffes zur Herstellung einer Wärmflasche.

Wärmflaschen der oben genannten Art sind allgemein bekannt. Es wird dabei aber darauf hingewiesen, dass unter einer Wärmflasche nicht jeglicher mit Flüssigkeit befüllbarer Behälter zu verstehen ist, sondern ausschließlich eine Wärmflasche, deren bestimmungsgemäßer Gebrauch zur Wärmebehandlung des menschlichen oder tierischen Körpers dient. Im ausländischen Sprachgebrauch wird der Begriff Wärmflasche mit bouillotte (FR), borsa (IT), termofor (PL), botija (PT), bolsa de aqua caliente (ES) übersetzt. Es wird diesbezüglich beispielhaft auf die Patentschrift DE 10 2009 013 231 B4 des Anmelders verwiesen. Weiterhin ist es grundsätzlich auch bekannt, solche Wärmflaschen mit einem Überzug auszustatten, um einen zu schnellen Wärmeübertrag von der Wärmflasche auf die Haut eines menschlichen Körpers zu verlangsamen und damit Verbrennungen zu verhindern. Außerdem wird der direkte und teilweise unangenehme Kontakt der Haut mit einem Kunststoff der Wärmflasche vermieden, durch den auch eine unangenehme Schweißbildung auf der Haut entstehen kann.

Weiterhin ist aus der Druckschrift DE 600 32 241 T2 ein Faser/Kunststoff-Verbundwerkstoff bekannt, wobei in der Beschreibung neben einer großen Vielzahl anderer Anwendungen erwähnt wird, dass aus diesem Verbundwerkstoff auch eine Wärmflasche herstellbar ist. Ebenso wird neben einer großen Anzahl weiterer Additive auch ein Biozid als Zusatz zu dem dort bereitgestellten Verbundwerkstoff erwähnt.

Aus der Druckschrift US 2,072,564 ist außerdem ein Stoffüberzug für eine Wärmflasche bekannt, der allerdings nicht zur Einmalverwendung ausgebildet ist.

Der Erfinder hat erkannt, dass bei der Verwendung der gleichen Wärmflasche zur Behandlung unterschiedlicher Personen beziehungsweise auch der gleichen Person hygienische Probleme entstehen können. Grundsätzlich ist es zwar möglich, die aus Kunststoff hergestellten Wärmflaschen an der außenliegenden Oberfläche vor und/oder nach einer Benutzung zu reinigen und zu desinfizieren, allerdings wird dies in der Praxis nur bedingt durchgeführt.

Es ist daher Aufgabe der Erfindung, eine Wärmflasche und eine Ausstattung einer Wärmflasche zu finden, welche verhindert, dass Keime, also Pilze, Bakterien und/oder Viren, bei serieller Verwendung durch mehrere Personen von Person zu Person übertragen werden. Außerdem soll auch verhindert werden, dass sich auf den Oberflächen der Wärmflasche Keimnester bilden beziehungsweise darüber Keime übertragen werden.

Diese Aufgabe wird durch die Merkmale der unabhängigen Patentansprüche gelöst. Vorteilhafte Weiterbildungen der Erfindung sind Gegenstand untergeordneter Ansprüche.

Der Erfinder hat weiterhin erkannt, dass es einerseits möglich ist, die aus Kunststoff, insbesondere aus Thermoplast, hergestellte Wärmflasche so auszugestalten, dass zumindest die mit der Umgebung, insbesondere der zu behandelnden Person, in Kontakt stehende Schicht mit einem biozid wirkenden Additiv oder einer biozid wirkenden Substanz versehen wird. Die biozide Wirkung erzeugt bestenfalls eine Abtötung der gegebenenfalls auf der Oberfläche der Wärmflasche befindlichen Keime oder zumindest eine Reduktion dieser Keime. Auch wird den Keimen der Nährboden für eine Weiterentwicklung entzogen.

Zum Zwecke der Klarstellung wird darauf hingewiesen, dass eine Substanz als biozid im Sinne dieser Erfindung definiert wird, wenn sie im engeren Sinne zumindest als bakterizid (= gegen Bakterien wirkend), akarizid (= gegen Milben wirkend), algizid (= gegen Algen wirkend), fungizid (= gegen Pilze wirkend), mikrobizid (= gegen Keime wirkend) und/oder viruzid (= gegen Viren wirkend) bezeichnet werden kann.

Alternativ oder ergänzend kann die Wärmflasche auch mit einem Einmalüberzug versehen werden, der nach jeder Benutzung entfernt und vor einer Benutzung bei einer anderen Person neu angebracht werden kann. Hierdurch wird verhindert, dass Keime von einer zur anderen Person weitergegeben werden.

Demgemäß schlägt der Erfinder eine Wärmflasche vor, welche einen über eine verschließbare Einfüllöffnung mit einer Flüssigkeit befüllbaren Hohlkörper aus einem Kunststoff, der kein Verbundstoff aus Fasern oder Kunststoff ist, mit einer Innenseite und einer Außenseite aufweist, wobei erfindungsgemäß zumindest das die Außenseite bildende Material der Wärmflasche mindestens eine biozid wirkende Substanz aufweist. Vornehmlich kann eine solche - grundsätzlich bekannte - Substanz als Additiv dem Kunststoff bei der Produktion beigefügt werden.

Grundsätzlich ist es damit ausreichend, wenn ausschließlich eine die Außenseite der Wärmflasche bildende Lage oder der die Außenseite bildende Anteil mindestens eine biozid wirkende Substanz aufweist. Entsprechend ist dann die die Innenseite bildende Lage beziehungsweise der die Innenseite bildende Bereich frei von bioziden Substanzen.

Unter bestimmten Bedingungen kann es jedoch auch günstig sein, wenn nicht nur die Außenflächen der Wärmflasche sondern auch die Innenflächen mindestens eine biozid wirkende Substanz aufweisen. Damit wird zusätzlich verhindert, dass sich in dem oft feuchten Innenraum des Hohlkörpers der Wärmflasche Keime ansammeln oder sich dort vermehren. Wird dem Kunststoff, aus dem die Wärmflasche hergestellt wird, eine entsprechende biozide Substanz mit Hilfe eines diese Substanz enthaltenden Additivs zugefügt, so befindet sich diese Substanz in allen Bereichen der aus Kunststoff hergestellten Wärmflasche. Damit sind zusätzlich zu den Außenflächen auch die Innenflächen der Wärmflasche biozid ausgeführt und verhindern einen Keimwachstum und/oder eine dauerhafte Keimablagerung auf allen Oberflächen.

Falls der Verschluss der Wärmflasche ebenfalls aus Kunstsoff besteht, kann dieser Kunststoff ebenso mit einer biozid wirkenden Substanz versehen werden.

Die Wärmflasche kann vorzugsweise mit einem den Hohlkörper bildenden Kunststoff ausgestattet sein, der zumindest einen der thermoplastischen Kunststoffe der folgenden Liste aufweist: Polyethylen (PE), Polyvinylchlorid (PVC), thermoplastisches Elastomer (TPE), Polyamid (PA), Polypropylen (PP).

Weiterhin kann bei der erfindungsgemäßen Wärmflasche die biozid wirkende Substanz zumindest eine der Substanzen der folgenden Liste enthalten: Silber (Ag); Kupfer (Cu); Zink (Zk); Metalloxide, insbesondere Wolframoxid, Molybdänoxid; antibakteriell wirkende organische Verbindungen, insbesondere Guanidin-Derivate; insbesondere können als Additiv auch die Produkte aus der VAH-Liste (VAH = Verbund für Angewandte Hygiene e.V.) verwendet werden, die unter der Internetadresse www.vah-online.de zu finden ist. Auf dieser Seite sind verschiedenste als Additive für Kunststoffe geeignete Substanzen angegeben, wobei deren besonderes Wirkungsspektrum, zum Beispiel fungizid, bakterizid oder viruzid, nach Bedarf gewählt werden kann.

Der Erfinder schlägt weiterhin vor, die eingangs beschriebene Wärmflasche oder eine sonstige erfindungsgemäße Wärmflasche mit einem für Krankheitserreger dichten Einmalüberzug auszustatten. Hierbei ist es wesentlich, dass der Einmalüberzug nicht einfach nur aus einem Gewebe oder Vlies besteht, da dieses in der Regel durchgängig für Keime ist und auf diese Weise Keime an die Oberfläche der Wärmflasche gelangen. Der erfindungsgemäße Einmalüberzug muss keimdicht beziehungsweise keimundurchlässig ausgestaltet sein. So wird einerseits verhindert, dass aufgebrachte Keime den Überzug durchdringen und sich auf der Wärmflasche selbst festsetzen, andererseits wird durch den Wechsel des Einmalüberzugs nach jedem Gebrauch vermieden, dass eine Übertragung gegebenenfalls darauf vorhandener Keime von Person zu Person stattfindet.

Hierfür eignet sich insbesondere ein Einmalüberzug, der mehrlagig ausgebildet ist. Dabei kann die nach Außen gewandte Lage aus einem gewebe- oder vliesartigem Material gebildet werden, so dass ein angenehmes Hautgefühl bei der Auflage auf den menschlichen Körper entsteht. Besonders bevorzugt kann dafür ein preisgünstig hergestelltes, Papiervlies verwendet werden.

Zur Abdichtung des Einmalüberzuges gegen Keime kann eine weitere, vorzugsweise innere, Lage verwendet werden, welche als PE-Folie oder PE-Beschichtung ausgebildet ist.

Alternativ besteht im Rahmen der Erfindung auch die Möglichkeit, die Keimabdichtung dadurch zu erreichen, dass mindestens eine Lage des Einmalüberzuges mit einer biozid wirkenden Substanz getränkt wird.

Günstig ist es außerdem, wenn der Überzug in seiner Kontur der Kontur der Wärmflasche angepasst ist. Insbesondere sollte durch die angepasste Kontur Raum für eine Einfüllöffnung entstehen.

Zum keimdichten Verschluss der Wärmflasche wird vorgeschlagen, dass der Überzug an einer Kante, an der sich keine Einfüllöffnung der Wärmflasche befindet, geöffnet ist, wobei die Öffnung weitgehend erregerdicht verschließbar ausgebildet ist. Beispielsweise können im Verschlussbereich Klebestreifen angebracht werden, so dass der Überzug mehrfach gefaltet verschlossen werden kann. Hierfür eignet sich ebenso eine Klemme oder eine dauerhaft biegbare Metalleinlage.

Weiterhin kann der Überzug auch eine Lage aus biozider Substanz oder eine mit biozider Substanz getränkte Lage aufweisen.

Vorteilhaft kann die biozide Substanz vornehmlich bakterizid, fungizid oder viruzid oder eine Kombination davon sein.

Entsprechend wird erfindungsgemäß auch ein Überzug für eine Wärmflasche vorgeschlagen, welcher zur einmaligen Verwendung ausgebildet ist. Dieser kann mehrlagig ausgebildet sein, wobei die nach Außen gewandte Lage aus einem gewebe- oder vliesartigem Material, insbesondere einem Textilgewebe oder Papiervlies, und mindestens eine, vorzugsweise die innere Lage als PE-Folie oder PE-Beschichtung ausgebildet ist.

Darüber hinaus ist es vorteilhaft, wenn der Überzug in seiner Kontur der Wärmflasche angepasst ist.

Günstig ist es außerdem, wenn der Überzug an einer Kante, an der sich keine Einfüllöffnung der Wärmflasche befindet, geöffnet ist, um den Überzug über eine Wärmflasche zu stülpen, wobei die Öffnung weitgehend erregerdicht verschließbar ausgebildet sein sollte. Hierzu können beispielsweise Klebestreifen, eine Klemme oder eine dauerhaft biegbare längliche Metalleinlage verwendet werden.

Im Übrigen wird im Rahmen der Erfindung auch die Verwendung eines mit einer biozid wirkenden Substanz versetzten Kunststoffes zur Herstellung einer Wärmflasche vorgeschlagen.

Ebenso wird auch die Verwendung einer biozid wirkenden Substanz zur Anreicherung mindestens einer Lage, vorzugsweise ausschließlich der äußeren Lage, eines mehrlagigen Überzugs einer Wärmflasche vorgeschlagen.

Es wird explizit darauf hingewiesen, dass der hier verwendete Begriff einer "Wärmflasche" sich ausschließlich auf einen Gegenstand bezieht, dessen bestimmungsgemäßer Gebrauch ausschließlich auf die Wärmebehandlung des menschlichen oder tierischen Körpers ausgerichtet ist beziehungsweise dazu dient. Beliebige andere Gefäße mit anderen Zweckbestimmungen zählen nicht zu dieser Erfindung.

Weiterhin wird klargestellt, dass das Vorliegen einer biozid wirkenden Substanz im Kunststoff bedeutet, dass eine solche Substanz im Kunststoff tatsächlich eine biozide Wirkung entfalten muss. Nicht zur Erfindung sollen Substanzen, insbesondere Elemente, zählen, die zwar in reiner Form biozid wirken, jedoch in der Molekularstruktur eines Kunststoffes eingebaut keine biozide Wirkung entfalten. Ein Beispiel hierfür ist das im PVC chemisch eingebaute Chlor. Chlor an sich wirkt zwar biozid, allerdings nicht in der Molekülstruktur des PVC, wo das Cl-Atom lediglich in dem Molekül (C₂H₃Cl)ₙ gebunden vorkommt.

Im Folgenden wird die Erfindung anhand der bevorzugten Ausführungsbeispiele mit Hilfe der Figuren näher beschrieben, wobei nur die zum Verständnis der Erfindung notwendigen Merkmale dargestellt sind. Es zeigen im Einzelnen:
- FIG 1a:: Wärmflasche, hergestellt aus Thermoplast mit biozid wirkendem Additiv;
- FIG 1b:: andere Wärmflasche, hergestellt aus Thermoplast mit biozid wirkendem Additiv;
- FIG 2:: Wärmflasche mit daneben liegendem Einmalbezug;
- FIG 3:: Einmalbezug mit darin eingeschlossener Wärmflasche;
- FIG 4:: Querschnitt durch eine Wärmflasche mit Einmalbezug.

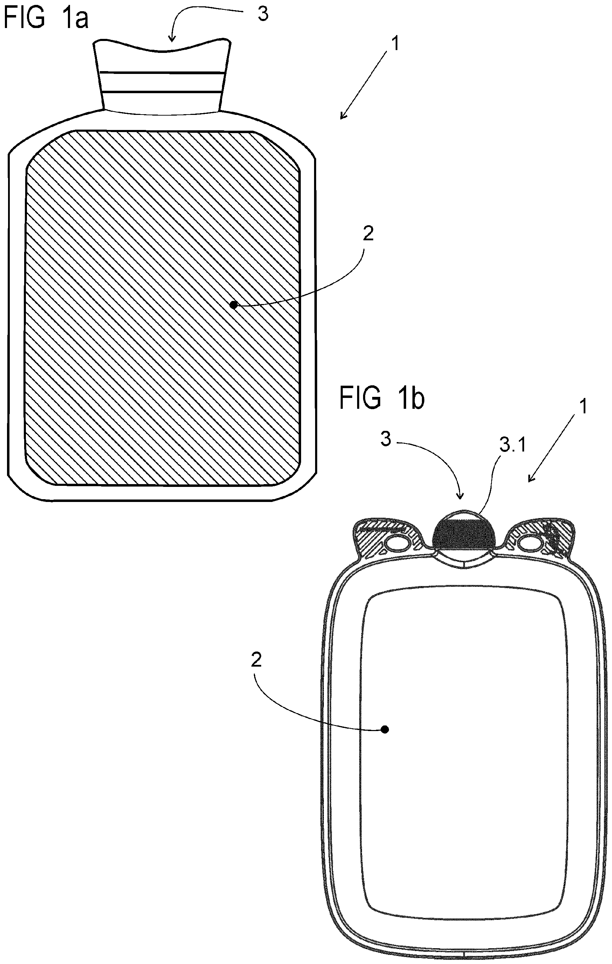

Die **Figuren 1a und 1b** zeigen jeweils eine beispielhafte Wärmflasche 1 in einer an sich bekannten Form und Herstellungsweise mit einem Hohlkörper 2 und einer mit einem Verschluss 3.1, der allerdings nur in der Ausführung der Figur 1b sichtbar ist, verschließbaren Einfüllöffnung 3, die stellvertretend für alle bekannten Wärmflaschen aus Kunststoff stehen soll, allerdings verfügt der für diese Wärmflasche verwendete Kunststoff zumindest an den nach Außen freien Flächen über ein biozides Additiv, wie es oben beschrieben ist. Ein solches biozides Additiv kann beispielsweise in feiner Körnung dem Kunststoff, mit dem die Wärmflasche produziert wird, beigefügt werden.

Es besteht allerdings auch die Möglichkeit, die Oberfläche der Form, in der die Wärmflasche geformt wird, mit einer bioziden Substanz zu belegen, so dass diese Belegung sich beim Herstellungsprozess mit der Oberfläche des Kunststoffmaterials der Wärmflasche verbindet. Eine solche Belegung kann beispielsweise durch entsprechend aufgetragenes Pulver, Gel oder Flüssigkeit bestehen. Es ist allerdings auch möglich, zum Beispiel biozides Material, wie beispielsweise Kupfer, zu einem dünnen und engmaschigen Geflecht zu verarbeiten und ein solches Geflecht mit der Oberfläche der Wärmflasche so zu verbinden, dass zumindest noch ein Teilbereich des Geflechtes unmittelbar mit der Außenseite Kontakt hat und dort seine biozide Wirkung entfalten kann.

Die in den Figuren 1a und 1b gezeigten Hohlkörper 2 der Wärmflaschen 1 wurden erfindungsgemäß mit einem thermoplastischen Elastomer (TPE) hergestellt. Beim Herstellungsprozess liegt die Kunststoffrohsubstanz als Granulat vor, das durch Erhitzen plastisch wird und letztlich in die Form der Wärmflasche gebracht wird. Diese granulatförmige Kunststoffrohsubstanz wird dann mit einem meist ebenso granulatförmigem Additiv möglichst gleichmäßig vermischt. Ein solches Additiv kann in einer bevorzugten Ausgestaltung aus einem Ethylen-Vinylacetat-Copolymere (= EVA oder EVAC) bestehen, in das wiederum die biozid wirkende Substanz, beispielsweise Zink-Pyrithion eingebettet ist. Mit dieser bioziden Ausstattung des gesamten Kunststoffes des Hohlkörpers 2 einschließlich des gegebenenfalls auch vorhandenen und dazugehörenden Kragens um die Einfüllöffnung 3, wie er in der Figur 1a gezeigt ist, wird der Hohlkörper 2, vorzugsweise bezüglich der außenliegenden Oberflächen, optional aber auch einschließlich aller innen- und außenliegenden Oberflächen, mit der bioziden Substanz durchsetzt. Entsprechend wird eine Keimbildung und Keimfestsetzung auf diesen Oberflächen verhindert. In gewissem Maße wird bei einer Verwendung der bioziden Substanz auch in dem Material, das die Innnenfläche bildet auch vermieden, dass sich im Wasser, welches über längere Zeit in der Wärmflasche steht, Keime vermehren.

Besteht der Verschluss 3.1 der Wärmflasche 1 ganz oder teilweise aus Kunststoff, so kann dieser Kunststoff ebenso mit einer biozid wirkenden Substanz versetzt werden, wie der Kunststoff des Hohlkörpers 2 der Wärmflasche 1. Es wird allerdings darauf hingewiesen, dass der Hohlkörper 2 und der Verschluss 3.1 meist aus unterschiedlichen Kunststoffen bestehen.

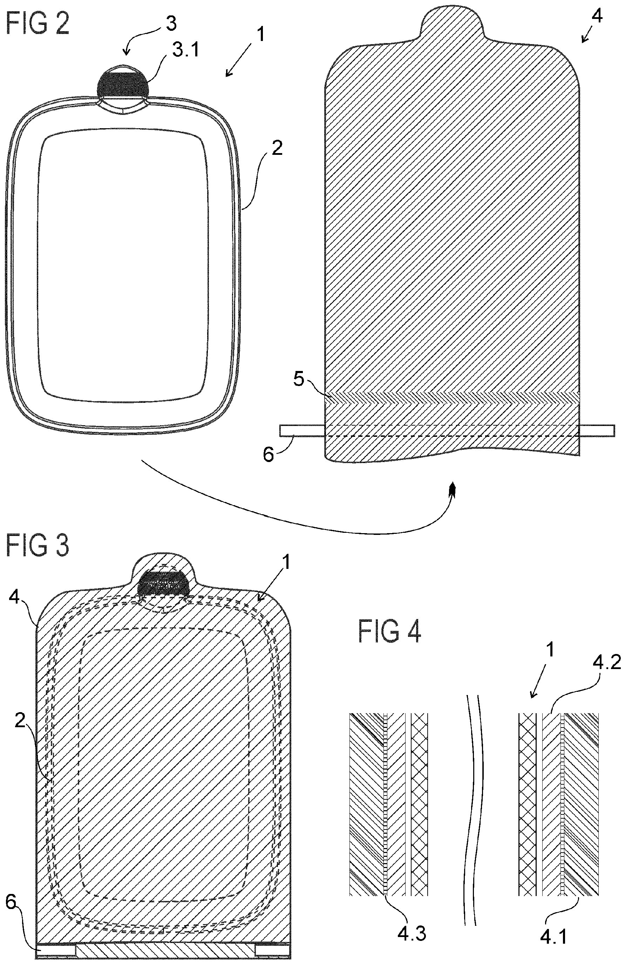

Eine andere alternative Ausstattung der Wärmflasche 1 oder auch eine ergänzende Ausstattung einer an sich schon biozid ausgestalteten Wärmflasche 1 ist in den Figuren 2 und 3 dargestellt. Die **Figur 2** zeigt die Wärmflasche 1 aus Kunststoff mit dem Hohlkörper 2 und der mit einer, durch einen Verschluss 3.1 verschlossenen, Einfüllöffnung 3. Rechts daneben ist ein Einmalbezug 4 dargestellt, der in seiner Kontur weitgehend an die Kontur der Wärmflasche 1 angepasst ist. Insbesondere weist der Einmalbezug 4 an der oben liegenden kurzen Seite eine Ausbuchtung auf, die bei eingesteckter Wärmflasche 1 sich um die Einfüllöffnung 3 mit dem darauf befindlichen Verschluss 3.1 anformt.

Der Einmalbezug 4 ist nach unten an seiner kurzen Seite offen, so dass durch diese Öffnung die bereits zuvor mit temperierter Flüssigkeit gefüllte und verschlossene Wärmflasche 1 eingesteckt werden kann. Weiterhin verfügt die Unterseite über einen quer verlaufenden Klebestreifen 5, welcher gegebenenfalls mit einer Schutzfolie überzogen sein kann, die bei Bedarf entfernt wird. Endständig im Bereich der Öffnung des Einmalbezuges ist außerdem ein Stabilisierungsstreifen 6 angeordnet, welche beispielhaft aus leicht biegbarem Metall, verformbarem Kunststoff oder einem festen Gewebe bestehen kann. Dieser Stabilisierungsstreifen 6 dient dazu, auf einfache Weise das untere Ende des Einmalbezugs 4 einrollen oder einfalten zu können, um den Einmalbezug 4 mit der darin befindlichen Wärmflasche 1 keimdicht abzuschließen. Unterstützt vom Klebestreifen 5 und/oder durch Einklappen der überstehenden Teile des Stabilisierungsstreifens 6 bleibt der Einmalbezug 4 auch bei einer Benutzung der darin befindlichen Wärmflasche 1 sicher und keimundurchlässig verschlossen.

Die **Figur 3** zeigt den Einmalbezug 4 mit darin befindlicher Wärmflasche 1.

Um auch bei Verwendung des Einmalbezuges 4 ein angenehmes Hautgefühl für den Nutzer zu gewährleisten, jedoch gleichzeitig eine Keimundurchlässigkeit des Einmalbezuges 4 sicherzustellen, ist dieser in bevorzugter Weise zweilagig ausgestaltet, wobei die äußere Lage aus einem Textilgewebe oder Papiervlies besteht und die innere Lage durch eine PE-Beschichtung oder PE-Folie gebildet wird.

Die **Figur 4** zeigt einen Querschnitt durch eine Wärmflasche 1 mit einem keimdichten Einmalbezug 4 und verdeutlicht einen erfindungsgemäßen Aufbau des Einmalbezuges 4. Dieser besteht aus einer äußeren hautfreundlichen Lage 4.1, hier aus einem Papiervlies oder einem Textilgewebe, und einer inneren keimdichten Lage 4.2, hier aus einer PE-Lage, wobei beide Lagen 4.1, 4.2 über eine dünne Klebeschicht 4.3 miteinander verbunden sind. Alternativ zur Klebeschicht 4.3 kann auch die keimdichte Lage 4.2 unmittelbar bei der Herstellung mit der hautfreundlichen Lage 4.1 verbunden werden, so dass beide Materialien ohne Zwischenschicht auskommen.

Mit einem solchen Einmalbezug ist es nun möglich, auch nicht aus biozidem Material hergestellte Wärmflaschen unter Auswechselung des Einmalüberzuges nacheinander für mehrere Personen zu verwenden und dabei trotzdem die hygienischen Voraussetzungen zu schaffen, die eine Keimübertragung zwischen den Personen verhindert.

Zum Rahmen der Erfindung zählt auch die Ausgestaltung des Einmalüberzuges, bei der mindestens eine der Lagen, vorzugsweise die äußere Lage, mit einem Biozid getränkt ist.

Insgesamt wird also mit dieser Erfindung eine Wärmflasche vorgeschlagen, welche zumindest teilweise aus einem Kunststoff hergestellt ist, welcher mindestens ein Biozid aufweist. Weiterhin betrifft die Erfindung einen Überzug einer Wärmflasche, der zur einmaligen Verwendung ausgebildet ist. Außerdem wird auch die Verwendung eines Kunststoffes mit einem Additiv mit biozider Wirkung zur Herstellung einer Wärmflasche vorgeschlagen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen. Insbesondere beschränkt sich die Erfindung nicht auf die nachfolgend angegebenen Merkmalskombinationen, sondern es können auch für den Fachmann offensichtlich ausführbare andere Kombinationen und Teilkombinationen aus den offenbarten Merkmalen gebildet werden. Ebenso liegt es im Rahmen der Erfindung, eine mechanische Umkehr der Funktionen der einzelnen mechanischen Elemente der Erfindung zu bewirken.

### Bezugszeichenliste

- 1: Wärmflasche
- 2: Hohlkörper
- 3: Einfüllöffnung
- 3.1: Verschluss
- 4: Einmalbezug
- 4.1: äußere Lage
- 4.2: innere Lage
- 4.3: Klebeschicht
- 5: Klebestreifen
- 6: Stabilisierungsstreifen

## Patentansprüche

1. Wärmflasche (1), aufweisend einen über eine verschließbare Einfüllöffnung (3) mit einer Flüssigkeit befüllbaren Hohlkörper (2) aus einem Kunststoff, der kein Verbundstoff aus Fasern oder Kunststoff ist, mit einer Innenseite und einer Außenseite, **dadurch gekennzeichnet, dass** zumindest das die Außenseite bildende Material der Wärmflasche (1) mindestens eine biozid wirkende Substanz aufweist.

2. Wärmflasche (1) gemäß dem voranstehenden Patentanspruch 1, **dadurch gekennzeichnet, dass** ausschließlich eine die Außenseite des Hohlkörpers (2) der Wärmflasche (1) bildende Lage oder der die Außenseite bildende Anteil mindestens eine biozid wirkende Substanz aufweist.

3. Wärmflasche (1) gemäß dem voranstehenden Patentanspruch 1, **dadurch gekennzeichnet, dass** der gesamte Kunststoff des Hohlkörpers (2) der Wärmflasche (1) mindestens eine biozid wirkende Substanz aufweist.

4. Wärmflasche (1) gemäß einem der voranstehenden Patentansprüche 1 bis 3, **dadurch gekennzeichnet, dass** die Wärmflasche (1) einen Verschluss (3.1) aufweist, der zumindest teilweise aus Kunststoff besteht, wobei auch dieser Kunststoff mindestens eine biozid wirkende Substanz aufweist.

5. Wärmflasche (1) gemäß dem Oberbegriff des Patentanspruches 1 oder gemäß einem der voranstehenden Patentansprüche, **dadurch gekennzeichnet, dass** die Wärmflasche (1) von einem Krankheitserreger abweisenden Überzug (4) umgeben ist.

6. Wärmflasche (1) gemäß dem voranstehenden Patentanspruch 5, **dadurch gekennzeichnet, dass** der Überzug (4) gemäß einem der nachfolgenden Ansprüche betreffend einen Überzug einer Wärmflasche ausgebildet ist.

7. Überzug (4) einer Wärmflasche (1), **dadurch gekennzeichnet, dass** der Überzug (4) zur einmaligen Verwendung ausgebildet ist.

8. Überzug (4) gemäß dem voranstehenden Patentanspruch 7, **dadurch gekennzeichnet, dass** der Überzug (4) mehrlagig ausgebildet ist.

9. Überzug (4) gemäß dem voranstehenden Patentanspruch 8, **dadurch gekennzeichnet, dass** die nach Außen gewandte Lage (4.1) aus einem vliesartigem Material, vorzugsweise einem Papiervlies, ausgebildet ist.

10. Überzug (4) gemäß einem der voranstehenden Patentansprüche 7 bis 9, **dadurch gekennzeichnet, dass** der Überzug für Krankheitserreger undurchdringbar ausgebildet ist.

11. Überzug (4) gemäß dem voranstehenden Patentanspruch 10, **dadurch gekennzeichnet, dass** die nach Innen gewandte Lage (4.2) als PE-Folie oder PE-Beschichtung ausgebildet ist.

12. Überzug (4) gemäß einem der voranstehenden Patentansprüche 7 bis 11, **dadurch gekennzeichnet , dass** der Überzug (4) in seiner Kontur der Wärmflasche (1) angepasst ist.

13. Überzug (4) gemäß einem der voranstehenden Patentansprüche 7 bis 12, **dadurch gekennzeichnet, dass** der Überzug (4) an einer Kante, an der sich keine Einfüllöffnung (3) der Wärmflasche (1) befindet, geöffnet ist, um den Überzug (4) über eine Wärmflasche (1) zu stülpen, wobei die Öffnung des Überzugs (4) weitgehend erregerdicht verschließbar ausgebildet ist.

14. Überzug (4) gemäß einem der voranstehenden Patentansprüche 7 bis 13, **dadurch gekennzeichnet, dass** der Überzug (4) eine Lage, vorzugsweise eine äußere Lage, aus biozider Substanz oder eine mit biozider Substanz getränkte Lage (4.1) aufweist.

15. Verwendung eines mit einer biozid wirkenden Substanz versetzten Kunststoffes zur Herstellung einer Wärmflasche (1).

16. Verwendung einer biozid wirkenden Substanz zur Anreicherung mindestens einer Lage, vorzugsweise ausschließlich einer äußeren Lage (4.1), eines mehrlagigen Überzugs (4) einer Wärmflasche (1).
